# EUROPÄISCHE PATENTSCHRIFT

(11) **EP 1 152 714 B1**
(45) Veröffentlichungstag und Bekanntmachung des Hinweises auf die Patenterteilung: **18.06.2003**
(21) Anmeldenummer: 00906170.6
(22) Anmeldetag: 01.02.2000
(51) Int. Cl.: A61F 2/16

(54) **INJEKTOR ZUM IMPLANTIEREN EINER GEFALTETEN INTRAOKULARLINSE, BEHÄLTER ZUM LAGERN UND TRANSPORTIEREN DES INJEKTORS SOWIE VERFAHREN ZUM GEFALTETEN AUSBRINGEN DER LINSE**
INJECTOR FOR IMPLANTING A FOLDED INTRAOCULAR LENS, CONTAINER FOR STORING AND TRANSPORTING THE INJECTOR AND METHOD FOR EJECTING THE LENS IN A FOLDED STATE
INJECTEUR POUR IMPLANTER UN CRISTALLIN ARTIFICIEL PLIE, CONTENANT POUR STOCKER ET TRANSPORTER LEDIT INJECTEUR, ET PROCEDE POUR APPLIQUER LEDIT CRISTALLIN EN LE PLIANT

(30) Priorität: 03.02.1999 DE 19904220
(43) Veröffentlichungstag der Anmeldung: 14.11.2001
(73) Patentinhaber: BINDER, Helmut, 60322 Frankfurt am Main (DE)
(72) Erfinder: BINDER, Helmut, 60322 Frankfurt am Main (DE)
(74) Vertreter: Petra, Elke, Dipl.-Ing.
(86) Internationale Anmeldenummer: DE0000262
(87) Internationale Veröffentlichungsnummer: WO00045746

(56) Entgegenhaltungen:
- WO-A-95/07059
- WO-A-95/13022
- WO-A-98/05281
- US-A- 5 123 905
- US-A- 5 190 552

## Beschreibung

Die Erfindung bezieht sich auf einen Injektor zum Implantieren einer (zeitweilig) gefalteten Intraokularlinse, mit dem die gefaltete Linse durch eine ca. 3 mm-Schnittöffnung im Auge in die Linsenkapsel des Auges einsetzbar ist, gemäß Oberbegriff des Patentanspruch 1.

In der Augenchirurgie ist es von großer Bedeutung, daß der Operationsschnitt, durch den der graue Star operiert und anschließend die erforderliche Kunstlinse implantiert wird, möglichst klein (ca. 3mm) ist, damit ein optimaler Heilungsprozeß garantiert und keine Naht erforderlich wird. Um Kunstlinsen mit dem erforderlichen Durchmesser von ca. 5 - 6mm implantieren zu können, müssen diese faltbar sein, damit sie durch den kleinen Schnitt von 3mm hindurchpassen.

Es sind unterschiedlichste Vorrichtungen zum Falten der Linsen und Einbringen der gefalteten Linse mit Hilfe einer Pinzette bekannt, und zudem Injektoren zum direkten Einbringen der Linsen in die Augen. Bei letzteren wird allgemein die im wesentlichen ungefaltet eingelegte Linse während des Vorschiebens im Injektor axial fortschreitend gefaltet, oder es wird eine bereits gefaltete Linse eingebracht und nur noch ausgeschoben.

So sind z. B. durch die EP 0 503 136 A1, EP 0 497 505 A1, EP 0 402 138 A1, WO 98/25548, WO 94/10912 und DE 40 39 119 C1 Vorrichtungen zum Falten einer Intraokularlinse in diversesten Ausführungsformen bekannt, durch die die Linsen jeweils entsprechend gefaltet bzw. vorgefaltet und danach über eine Pinzette oder über einen Injektor, in das Auge eingebracht werden. Dieser Vorgang ist relativ komplex und zudem durch die Handhabung mit der Pinzette auch unsicher, da die gefaltete Linse leicht aus der Pinzette herausrutschen kann.

Aus den DE 41 10 278 A1, DE 36 10 925 C2, WO 96/15743, EP 0 270 257 A1, EP 0 477 466 A1, WO 97/113 476, WO 97/15253 sind Linsen-Injektoren bekannt, bei denen zum Teil bereits vorgefaltete Linsen in die Aufnahmekammer einer am vorderen Ende des Injektors befestigbaren Einbring-Patrone eingebracht wird. Diese Patrone dient meist gleichzeitig als Aufnahmekammer für die Aufbewahrung und den Transport der Linse. Nach Aufsetzen der Patrone auf das Handstück des Injektorkörpers wird durch axiales Verschieben mittels eines Stößels die Linse durch den sich verjüngenden Kanal bis ans Austrittsende geschoben, wodurch die Linse gefaltet oder bzw. noch nachgefaltet wird. Diese bekannten Injektoren sind relativ komplex aufgebaut, aus vielen Einzelteilen und der Verwender muß zumindest zwei separate Teile zusammensetzen, bevor die Linse ausgeschoben werden kann.

Aus der WO 97/13476 ist ein Injektor bekannt, bei dem die Linse vorhergehend in einem Faltinstrument gefaltet und dann zusammen mit diesem radial in den Injektor eingelegt wird. Anschließend wird über einen Stößel die gefaltete Linse aus dem Faltgerät durch die sich verjüngende Ausbringhülle hindurchgeschoben, wodurch die Linse eine noch weitergehende Faltung erfährt. Es sind hier somit zwei Instrumente notwendig und zwar eines zum Vorfalten und eines zum Injizieren der' gefalteten Linse.

Aus der WO 98/05281 ist ein Injektor bekannt, dessen die Linse haltende Patrone an ihrem rückwärtigen Ende eine radial ausschwenkbare Klappe mit mittiger Halterippe aufweist, über welche die ungefaltete Linse in den Transportkanal radial eingedrückt und dort festgehalten wird. Eine echte Faltung der Linse erfolgt jedoch erst durch das Vorwärtsschieben der Linse durch den sich verjüngenden Transportkanal. Es ist hier somit ein relativ komplexer Aufbau mit entsprechend komplexer Handhabung vorhanden.

Auch beschreiben die WO 95/07059 und WO 95/13022 einen Injektor mit zylindrichem Transportkanal, in den eine ungefaltete Linse mit Hilfe einer eigenständigen. mit dem Injektorkörper nicht verbundenen Schieber-Rippe tangential so in den Transportkanal eingeschoben wird, daß sie sich in diesem einrollt. Ober einen ebenfalls separaten, zylindrischen, den Transportkanal ganz ausfüllenden Schieber wird die gerollte Linse dann ausgeschoben. Es sind somit jeweils zwei separate Teile in den Injektor einzuführen und die Linse gerollt auszubringen.

Zudem beschreibt die US 5,123,905 A einen lnjektor, mit einem zylindrischen Körper und darin koaxial vorgesehenen zylindrischen Transportkanal, der in einer konisch sich verjüngenden Ausbringspitze mündet. Im zylindrischen Injektorkörper ist ein relativ schmaler, radialer Einbringschlitz vorgesehen, durch den die über dem Einbringschlitz auf den Körpermantel aufgelegte Linse mit Hilfe einer eigenständigen Einbringrippe eingedrückt wird. Abgesehen davon, daß dieser bekannte Injektor nicht auch gleichzeitig für Transport und Aufbewahrung einer ungefalteten Linse dienen kann, ist das Einbringen der Linsen durch den sehr engen Einbringschlitz beschwerlich, ja unmöglich, da bekanntlich die Linsen praktisch inkompressibel sind. Auch füllt der ebenfalls als eigenständiger starr ausgelegte Schieber den gesamten Querschnitt des Transportkanals aus, so daß nach dem Einbringen der Linse die Einbringrippe entfernt werden muß. Zudem läuft der Transportkanal konisch sich verjüngend spitz zu, so daß die eingebrachte, in zylindrischer Form im Kanal befindliche Linse beim Ausschieben noch weiter zusammengerollt wird, was erhebliche Ausschubprobleme ergibt.

Schließlich zeigen die DE 43 03 051 A1 und die US 5,190,552 A einen Injektor, bei dem eine ungefaltete Linse über eine radiale Eingabeöffnung in den in diesem Abschnitt radial nach oben offenen Transportkanal eingelegt wird und zwar auf einen Linsen-Halteschlitten. Über eine Linsen-Verriegelungsstange, die axial über die eingelegte Linse hinwegführend geschoben wird, wird die Linse gegen Herausfallen festgehalten. Beim Ausschieben werden Haltestange und Schlitten zusammen durch einen zumindest am Anfang sich verjüngenden Transportkanal des Einbringrohres geschoben, wodurch die Linse, mittig weiterhin festgehalten durch die Haltestange, um diese Stange gefaltet wird. Auch hier ist ein relativ komplexer Aufbau und in Verbindung damit eine relativ komplexe Handhabung vorhanden. Zudem muß die Linse in ihrer auf dem Schlitten eingelegten Position während des Vorschiebens von der Verriegelungsstange angedrückt festgehalten werden, um durch die Stange nicht vom Schlitten abgeschoben zu werden.

Aufgabe der Erfindung ist es, einen Injektor oben genannter Gattung anzugeben, der einen möglichst einfachen Aufbau aufweist und eine sichere, sterile Handhabung während Aufbewahrung, Transport, Falten und Implantieren der Linsen erlaubt.

Diese Aufgabe wird erfindungsgemäß durch einen Injektor mit den Merkmalen des Patentanspruchs 1 gelöst.

Demgemäß ist der Transportkanal des einteilig ausgebildeten Injektorkörpers eine axial durchgehende Öffnung mit konstantem Querschnitt. Der Transportkanal ist somit durch den gesamten Injektor hindurch, also vom Schiebereintrittsende bis hin zum Linsen-Ausschiebende, mit einem durchgehend gleichen Querschnitt versehen, was eine herstellungsmäßige Vereinfachung darstellt. Zudem ist die Auflagefläche für die ungefaltete Linse radial in Einbringrichtung nach oben versetzt zum Transportkanal angeordnet. Hierdurch ist die Linse oberhalb des Transportkanais bzw. zumindest wesentlich beabstandet vom Boden des Tranportkanals, gleichzeitig jedoch unterhalb der oberen Fläche des Körpers aufliegend eingebracht. Schließlich ist die die ungefaltete Linse auf der Auflagefläche festhaltende Halterippe bzw. - stange eine radial ausladende, plattenförmige Faltrippe, die in Art eines Prägewerkzeug-Stempels angeordnet ist und durch die schlitzförmige Einbringöffnung bis weit in den Transportkanal radial bzw. quer zur Transportrichtung eindrückbar ist. Faltrippe und Körper wirken somit wie entsprechend ausgelegte Matritze und Patritze auf die zwischenliegende Linse ein und biegen diese mittig durch, sie gleichzeitig in den Transportkanal in fertig gefaltetem Zustand einschiebend. Die Linse braucht dann nur noch in diesem fertig gefalteten Zustand im Transportkanal durch den Schieber, der hier selbstverständlich ebenfalls eine Stange konstanten Querschnitts ist, vorwärtsgeschoben zu werden.

Der einstückige Injektorkörper kann in Weiterbildung des Erfindungsgedankens insgesamt einen Rechteck-Querschnitt aufweisen, d.h. sowohl der dickere Einbringund Falteteil als auch der dünnere Einführtri und auch der Transportkanal weisen jeweils einen Rechteck-Querschnitt auf. Alle diese Teile, nämlich Körperabschnitte und Transportkanal, können auch einen runden oder ovalen Querschnitt aufweisen oder der Halteteil kann rechteckig ausgebildet sein, während Einführrohr und Transpottkanal einen runden Querschnitt aufweisen.

Selbstverständlich ist der Transportkanal in seinem Querschnitt gemäß den Abmessungen der gefalteten Linse auszulegen. So muß die Kanalbreite ungefähr gleich der doppelten Linsendicke plus Faltrippendicke sein, während die Kanalhöhe zumindest gleich dem halben Linsendurchmesser ausgelegt sein sollte. Auch ist die Faltrippe in ihrer Breite im Verhältnis zu den sie umgebenden Flächen wie Transportkanalwänden, so ausgelegt, daß die Linsenhaptiken beim Falten und Verschieben nicht eingeklemmt und beschädigt werden.

Da die Dimensionen des Injektors insgesamt relativ gering sind, sind seine Bestandteile während der Betätigung teilweise ineinandergreifend realisiert, was durch die maximale Vereinfachung der Bestandteile möglich ist. Nach dem Falten der Linse durch Eindrücken der Faltrippe bis in den Transportkanal, verbleibt nämlich die Faitrippe in dem so eingedrückten Zustand auch während des Ausschiebens der gefalteten Linse, vorzugsweise eingerastet. Daher weist die querschnittskonstante Schiebestange an ihrer Oberseite eine Längsnut auf, die die Faltrippe während dem Längsverschieben mit leichter Schiebetoleranz U-förmlg umgreift. Zudem ist von Vorteil, wenn die vordere Seite des Schiebers eine der Form der gefalteten Linse angepaßte Ausbildung erhält, also eine ca. Viertel-Zylinder-Schweifung nach innen, wodurch ein gleichmäßiger Schiebedruck auf die Linse ausgeübt wird.

Um ein sicheres Handhaben des Injektors in seinen Rollen als Linsenhalte-, als Linsenfalt- und als Linseneinbringvorrichtung sicherzustellen, sind für die vier verschiedenen Arbeits-Endpositionen Rastier- bzw. Indexiervorkehrungen an Schieber, Faltklappe und Körper vorgesehen.

So sind für den Schieber drei Positionen vorgesehen, mit entsprechenden Positioniervorkehrungen, u.zw. eine erste Position zum Festlegen der Schieber-Positionen in hinter die radiale Eingabeöffnung zurückgezogene Stellung, eine zweite vorgerückte Position, in der die gefaltete Linse nahe zur vorderen Austragsöffnung des Einführrohres geschoben ist und schließlich eine dritte und letzte Position, mit bis zur Austrittsöffnung des Einführrohres reichender Schieber-Stirnfläche. Die beiden ersten Positionen können durch Indexierkugeln bzw. Noppen o.ä. Vorkehrungen realisiert sein, während die dritte Position auch durch Anschlagen des am äußeren, aus dem Injektorkörper herausragenden Ende des Schiebers vorgesehenen Druckplatte am Injektorkörper realisiert sein kann.

Erfindungsgemäß ist die vorzugsweise runde Auflagefläche für die ungefaltete Linse durch den Einbring- und Faltschlitz in zwei Teiiflächen getrennt, die horizontal, d.h. parallel zum Boden des Transportkanals und gleichzeitig zur Oberseite des Injektorkörpers ausgerichtet ist. Diese Teil-Auflageflächen können jedoch auch dachförmig nach innen in Richtung auf den Transportkanal geneigt ausgerichtet sein, vorzugsweise in einem Winkel von 30° zur Horizontalen bzw. zur Körperoberseite, bzw. in einer Neigung, die annähernd der Neigung des Linsenkörpers entspricht Hierdurch und durch zusätzliche große Übergangsradien zwischen Auflageflächeteilen und Faltschlitz ist eine optimale Faltbewegung möglich.

Von Vorteil ist, wenn knapp oberhalb der Auflagefläche längsseitige schmale Auflageleisten vorgesehen sind, über die die Linse leicht einschnappend auf die Auflagefläche eingebracht wird und die gleichzeitig als Auflageleisten für die an der Faitrippe bzw. am entsprechenden Klapphebel vorgesehene Anschlagleisten dienen. Somit wird durch diese Auflageleisten zugleich ein zumindest leichtes Festhalten der Linsen realisiert.

Die über die Einbringöffnung in und aus dem Transportkanal radial schwenkbare Fattrippe ist am vorderen Ende eines an der Körperoberseite schwenkbar angelenkten Klapphebels vorgesehen. Dabei kann an der Oberseite des Körper-Halteteils eine entsprechend breite Nut vorgesehen sein, in der der Klapphebel in eingeschwenktem bzw. eingeklapptem Zustand komplett eingelassen ist, ohne aus der Fläche herauszuragen. Um dann jedoch ein emeutes Ausklappen des Klapphebels zu ermöglichen, muß z.B. eine über der Faltrippe am Hebel vorgesehene Druckplatte, die die gesamte Körperbreite einnimmt o.ä. Vorkehrungen ein Angreifen an dem eingelassenen Hebel möglich sein. Der Hebel kann jedoch auch flach auf dem Körper aufliegend über entsprechende Gelenkohren am Körper angelenkt sein. Diese Gelenkohren des Körpers können dabei in bekannter Weise nach oben über einen schmalen Schlitz geöffnet sein, wodurch die Gelenkzapfen über diese Schlitze in die Lageröffnungen eindrückbar sind. Aber auch in der im Körper eingelassenen Ausführung des Hebels kann eine solche Schnappmontage des Scharniers vorgesehen sein.

Um über den um ca. 180° umklappbaren Hebel der Faltrippe die zwei Arbeitspositionen der Faltrippe festzulegen, sind an Hebel und Körper entsprechende Indexiereinrichtungen vorgesehen, durch die jeweils eine Hebel-Winkelposition beim aufliegenden Festhalten der ungefalteten Linse und beim eingedrückten Halten der gefalteten Linse festgelegt werden. Dadurch wird verhindert, daß in Festhalte- oder in Faltende-Position der Hebel nach oben abschwenkt oder einen zu geringen Druck im weiteren ausübt, so daß die Linse herausfällt bzw. sich verschiebt, wodurch deren korrektes Ausschieben nicht sichergestellt ist.

Gemäß einer Weiterbildung des Erfindungsgedankens ist zur Handhabungserleichterung und auch zur nachfolgend beschriebenen Fixierung des Injektors in einem Transportbehälter zu beiden Seiten des Körper-Halteteils ca. mittig je eine quer ausladende Griffplatte angebracht. Hierdurch kann, insbesondere beim letzendlichen Ausbringen der gefalteten Linse aus dem Injektor bzw. Einbringen dieser Linse in das Auge, mit dem Injektor in ungefähr gleicher Weise wie mit einer Injektionsspritze umgegangen werden.

Von besonderem Vorteil ist, wenn der aus nur drei Teilen, also aus Injektorkörper, Schieber und Klappe, bestehende Injektor insgesamt aus transparentem Kunststoff gefertigt ist, z.B. durch Spritzgießen, u.zw. so, daß die 3 Injektorbestandteile durch einfaches Ineinander- bzw. Aufeinanderdrücken zusammengesetzt bzw. zusammengehalten werden. Abgesehen davon, kann durch die durchsichtige Ausführungsweise des Injektors die jeweilige Lage der Linse genau verfolgt werden. Z.B. kann beobachtet werden, wie die ungefaltete Linse aufliegt, wie diese dann über die Faltrippe durch Eindrücken bis in den Transportkanal gefaltet und danach über den Schieber vor und dann ausgeschoben wird. Irgendwelche Fehllagen der Linse können dabei sogleich beobachtet werden, so daß sich der den Injektor Handhabende auf diese Gegebenheit in seiner weiteren Tätigkeit einstellen kann.

Selbstverständlich kann aus Fertigungsgründen der Injektorkörper zweiteilig ausgebildet sein und zwar mit einer zum Transportkanal parallelen Trennebene. Nach z.B. Spritzgußfertigen sind die beiden Körperteile zusammenzufügen und zu verkleben.

Auch kann erfindungsgemäß der Schieber zweiteilig ausgebildet sein, wobei er aus einem vorderen, längsverschieblichen Teil und einem rückwärtigen, drehbaren Teil zusammengesetzt ist. Der rückwärtige Teil weist ein Gewinde auf, das in einer Gewindebohrung des Halteteils geführt ist, um einen genau steuerbaren Vorschub des Schiebers und damit der Linse sicherzustellen. Über bekannte Mittel, wie Öffnung und Schnappbolzen an den aneinanderliegenden Stirnseiten der Schieberteile kann zudem der gemeinsame Rückzug des Gesamtschiebers erreicht werden.

Zum Lagern und Transportieren eines erfindungsgemäßen Injektors kann ein Aufnahmebehälter verwendet werden, der im wesentlichen aus einer Hülse mit Deckel besteht, in dem innen seitliche Halteplatten zum Positionieren des Injektors über dessen seitliche Griffplatten vorgesehen sind. Die Halteplatten besitzen je einen in Einführrichtung weisenden Widerhaken, die die Injek-tor-Griffplatten umgreifen und den Injektor an der Behälterinnenwand anliegend festhalten. Durch einen axialen Dom am Deckel, der an der rückwärtigen Stimfläche des Injektorkörpers bei aufgesetztem Deckel ansteht oder durch ein zwischen rückwärtiger Stimseite des Injektorkörpers und hinterer Druckplatte des Schiebers aufgebrachtes Abstand-Schlitzrohr und zudem die Anlage der rückwärtigen Stirnfläche der Schieber-Druckplatte an der Innenstimseite des Deckels, die mit sehr weich elastischem Kunststoff belegt sein kann, wird eine optimale Positionierung des Injektors im Behälter erreicht. So kann auch bei unvorsichtiger Handhabung des Behälters der Schieber nicht unbeabsichtigt über seine hintere, zurückgezogene Einrastposition hinweg im Tranportkanal vorwärtsgeschoben werden, wodurch die auf der Auflagefläche aufliegend ungefattete Linse abgestoßen und ausgeworfen oder gar beschädigt werden könnte.

Der Tranportbehälter oder zumindest dessen Aufnahmehülse kann ebenfalls aus transparentem Kunststoff in an sich bekannter Weise, z.B. durch Spritzgießen oder Blastechnik gefertigt sein, wodurch gut festgestellt werden kann, ob der Injektor - und damit die Linse - auch die richtige Position einnehmen.

Der Transportbehälter mit darin befindlichem Injektor kann mit einer zur Aufbewahrung und Lieferung von Faltlinsen bekannten, sterilen Transportflüssigkeit gefüllt sein. Dabei wird die Behälterhülse, nachdem der Injektor eingeführt wurde, in vertikaler, nach oben offener Stellung, mit der Flüssigkeit vollgefüllt. Beim Aufsetzen des Deckels verdrängt der Deckeldom und /oder der innere Deckelkegel Flüssigkeit, so daß keine Luft im geschlossenen Behälter verbleibt, wodurch eine sterile, die Linseneiastizität beibehaltende Aufbewahrung des Injektors sichergestellt ist.

Der Transportbehälter kann - wie dies bei Medikamenten und medizinischen Instrumenten allgemein üblich ist - seinerseits in einem Verpackungskarton eingelegt sein, in dem zudem ein Beipackzettel eingelegt ist, wodurch ein zusätzlicher Schutz für den Transportbehälter und damit des Injektors und schließlich der Linse selbst sichergestellt ist.

Beim gefalteten Ausbringen einer Intraokularlinse durch den erfindungsgemäßen lnjektor wird der die radiale Einbringöffnung im Injektorkörper verdeckende Klapphebel um ca. 180° zurückgeklappt, die Einbringöffnung freilegend, wonach in die am oberen Ende der Einbringöffnung vorgesehene Auflagefläche eine ungefaltete Linse ein- bzw. aufgelegt wird. Danach wird der Klapphebel emeut um annähemd 180° zurückverschwenkt, bis er mit der Unterseite seiner Faltrippe auf der Oberseite des Linsenkörpers aufsteht, möglichst unter Einrasten des Hebels in dieser Festhalteposition. In diesem Zustand wird der "geladene" Injektor dann in Gebrauch genommen oder in einen Transportbehälter eingeschoben und fixiert und durch Einlegen in einen Verpackungskarton usw. für spätere Verwendung aufbewahrt oder für den Transport bis zu weiteren Verwendungsstellen bereitgestellt.

Bei Inbenutzungsnahme während einer Implantations-Operation wird beim eventuell vorhergehend aus einem Behälter entnommene Injektor der Klapphebel z.B. durch Pressen auf dessen Druckplatte mit darunter befindlicher Faltrippe radial bis auf Anschlag eingedrückt, wodurch die Faltrippe die darunter befindliche Linse von der Auflagefläche durch den Faltschlitz in den Transportkanal drückt. Gleichzeitig liegt bzw. faltet sich die Linse längssymmetrisch elastisch um die Faltrippe und wird so in den gefalteten Einbringzustand versetzt. Danach wird der Schieber aus seiner zurückgesetzten Ruhe-Position in die zweite Position geschoben, wodurch die Linse in ihre Stellung nahe der Auswurföffnung im Transportkanal gebracht wird. So ist der Injektor einsatzbereit für die Implantation der in ihm befindlichen, gefalteten Linse.

Nachdem dann der kurze Operationsschnitt am Auge durchgeführt und der schmale Einführrohrteil des Injektors, dessen Spitze für die einfachere Einführung möglichst flach zugespitzt auslaufend ausgebildet sein sollte, in das Augeninnere, bis kurz über den Linsensack durch die Iris eingeführt wurde, wird der Injektorschieber weiter einwärts geschoben bis auf End-Anschlag, wodurch die Linse aus der Irjektorspitze in den Linsensack ausgeschoben wird. Danach wird der injektor zurückgezogen und entweder als Einwegteil weggeworfen oder als Mehrwegteil zur Wiederbestückung mit einer ungefalteten Linse aufbewahrt.

Nachfolgend wird die Erfindung anhand mehrerer Ausführungsbeispiele unter Bezug auf die Zeichnung näher beschrieben.

Es zeigen:
- Fig. 1:: eine Seitenansicht auf einen erfindungsgemäßen Injektor in erster Ausführung, mit eingelegter, ungefalteter Linse und aufliegender Halteklappe,
- Fig. 2:: eine Seitenansicht wie in Fig. 1, mit eingedrückter Klappe und dadurch gefalteter Linse,
- Fig. 3:: eine Draufsicht auf einen Injektor nach Fig. 1 und 2,
- Fig. 4:: einen Schnitt III-III aus Fig. 2, die Anordnung der einzelnen Teile zueinander klarer zeigend,
- Fig. 5:: eine Stimansicht gemäß Pfeil V aus Fig. 1,
- Fig. 6:: eine Seitenansicht auf einen Injektorkörper in zweiter Ausführung, mit rechteckigem Körper-Halteteil und rundem Einführrohr, Transportkanal und Schieber,
- Fig. 7:: eine Stimansicht nach Pfeil VII aus Fig. 6,
- Fig. 8:: einen Schnitt VIII-VIII aus Fig. 6,
- Fig. 9:: eine Draufsicht auf den Injektorkörper gemäß Fig. 6,
- Fig. 10:: eine Seitenansicht eines Klapphebels mit Faltrippen, zum Einsatz in Verbindung mit dem Injektorkörper nach Fig. 5 - 8,
- Fig. 11:: eine Draufsicht auf den Klapphebel nach Fig. 10,
- Fig. 12:: eine Seitenansicht auf einen Schieber zum Einsatz in Verbindung mit dem Injektorkörper nach Fig. 6 bis 9,
- Fig. 13:: eine Vorderansicht des Schiebers nach Fig. 12,
- Fig. 14:: eine Draufsicht auf den Schieber nach Fig. 12 und 13, die Nut zum Schieben entlang der Faltrippe zeigend,
- Fig. 15:: eine Seitenansicht im Maßstab 1:1 auf einen Injektor nach Fig. 6 bis 14,
- Fig. 16:: einen vertikalen Schnitt XVI-XVI aus Fig. 15, mit eingedrücktem Klapphebel, in leicht abgewandelter Form von Hebel und Auflagefläche.
- Fig. 17:: einen Längsschnitt durch einen im Transportbehälter und Verpackungskarton verpackten Injektor,
- Fig. 18:: eine Stirnansicht mit entferntem Boden von Behälter und Karton.

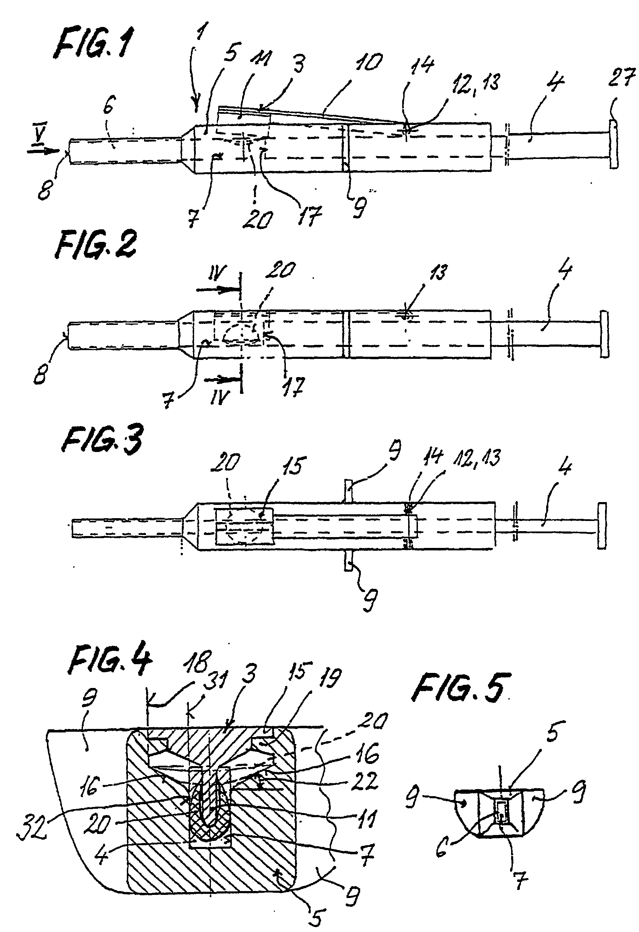

Wie insbesondere aus Fig. 1 ersichtlich ist, besteht ein erfindungsgemäßer lnjektor 1 prinzipiell aus einem Körper 2, an dem eine Klappe 3 verschwenkbar angelenkt ist und in dem längsverschieblich ein Schieber 4 angeordnet ist Der Injektor besteht somit lediglich aus drei einfachen Bauteilen und ist daher einfach herstell- und handhabbar.

Der einteilige Körper 2 besteht aus einem dickeren Einbring- und Halteteil 5 und einem wesentlich dünneren Einführrohr 6 und hat einen durchgehenden Transportkanal 7 mit konstantem Querschnitt. Beide Körperteile besitzen einen rechteckigen Querschnitt, wie aus Fig. 2 ersichtlich ist Die vordere Stimseite des Einführrohres 6 mit der Austragöffnung 8 ist senkrecht zur Längsachse des Injektors ausgebildet. Zu beiden Seiten des Körper-Halteteils 5 sind mittig je eine Griffplatte 9 seitlich ausladend befestigt, so daß der Injektor insbesondere beim Einbringen der Linse ins Auge in Art einer Injektionsspritze gehandhabt werden kann. Zudem dienen diese Griffplatten zur Fixierung des Injektors in einem Transportbehälter, der anhand weiterer Figuren nachfolgend genauer beschrieben wird.

Wie aus Fig. 1 bis 4 zu erkennen ist, besteht die Klappe 3 aus einem Hebel 10, der an der Unterseite seines vorderen Endes eine nach unten herausragende, plattenförmige Faltrippe 11 aufweist Der Klappenhebel 10 ist an seinem anderen Ende über seitlich herausragende Achsstummel 12, die auch aus Fig. 3 ersichtlich sind, an der Oberseite des Körper-Halteteils 2 in entsprechenden Lagerbohrungen 13 schwenkbar gelagert. Diese Lagerbohrungen 13 sind nach oben hin durch einen Schnappschlitz 14 offen, so daß zur Montage der Klappe 3 am Körper 1 die Achsstummel 12 durch den Schnappschlitz 14 lediglich in die Lagerbohrungen 13 eingedrückt werden müssen. Wie auch aus Fig. 4 gut erkennbar ist, ist die Klappe 3 an ihrem vorderen Ende mit einer breiteren Druckplatte 15 ausgestattet, an deren Unterseite die Faltrippe 11 vorgesehen ist. Wie aus Fig. 1 auch zu erkennen ist, ist die hier noch ungefaltete Linse 20 in der oberen Zone des Transportkanals 7 auf einer aus Fig. 4 ersichtlichen Auflagefläche 16 aufliegend gehalten bzw. angeordnet und wird durch die Unterseite der Faltrippe 11 gegen unbeabsichtigtes Herausfallen festgehalten.

In Fig. 2 ist die Klappe 3 in eingedrücktem Zustand darge-stellt, und es ist erkennbar, daß in diesem Zustand die Linse 20 auf die Hälfte gefaltet sich insgesamt im Transportkanal 7 befindet. Dabei befindet sich die Stimseite 17 des Stempels 4 in Austragrichtung gesehen hinter der Linse und kann durch Längsverschieben die Linse nach vome, in Richtung auf die Austrittsöffnung 8 im Transportkanal 7 vorwärtsschieben.

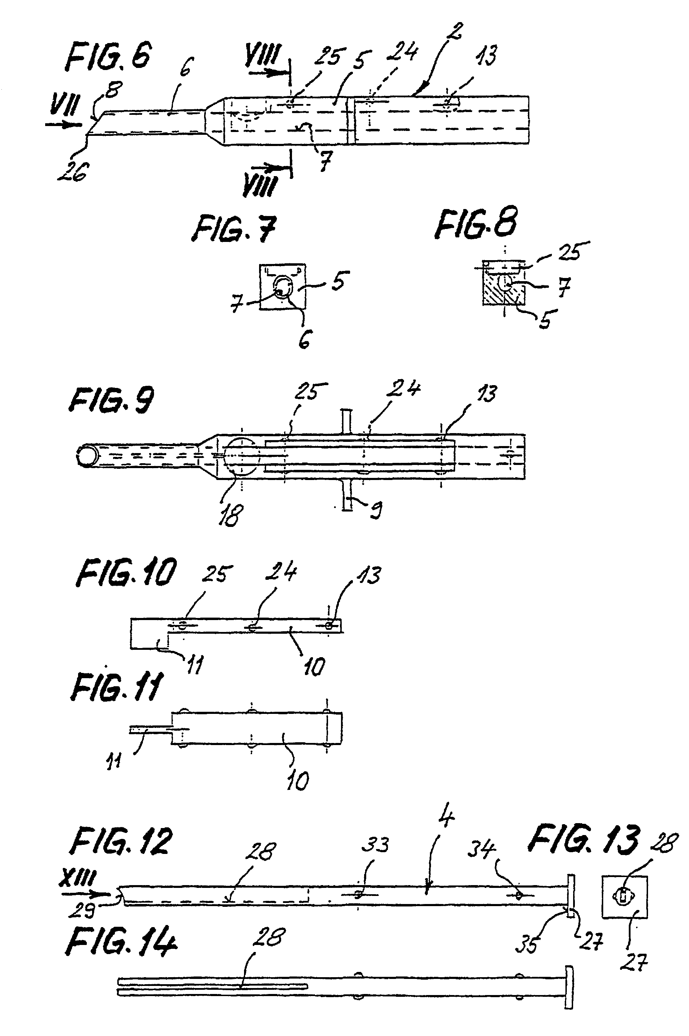

Aus Fig. 5 ist die rechteckige Ausführungsform des Injektorkörpers, mit Halteteil 5, Einführrohr 6 und Transportkanal 7 zu erkennen. Auch sind da die beiden Griffplatten 9 erkennbar, die hier seitlich annähernd viertelkreisförmig abgerundet sind, in Anpassung an die Form der Innenwand des nachfolgend noch näher beschriebenen runden Transportbehälters. Bei Verwendung eines rechteckigen Transportbehälters sind die Griffplatten selbstverständlich ebenfalls rechteckig ausgebildet.

In Fig. 4, die die Eindrücksituation der Klappe aus Fig. 3 genauer zeigt, ist die rechteckige Form des Körper-Haiteteils 5 gut zu ersehen. In diesem Halteteil 5 ist ein rechteckiger Schlitz vorhanden, der hier gleichzeitig - zumindest seine Unterseite betreffend - Teil des Transportkanals 7 ist, in dem der Schieber 4 längsverschieblich eingebracht ist An der Oberseite des Halteteils 5 ist eine Einbringöffnung 18 vorgesehen, die in Länge und Breite mindestens die Abmessung der Linse 20 aufweisen muß und rund sein kann, wie aus Fig. 5 ersichtlich ist. Bei der in Fig. 4 dargestellten Ausführungsform sind zu beiden Seiten der Einführöffnung 18 je eine Auflageleiste 19 vorgesehen, auf der sich die Druckplatte 15 in ihrer Faltbewegung nach unten abschließend abstützt. Die Leisten 19 dienen auch gleichzeitig einem groben Positionsfesthalten der Linse, die beim Einführen über diese Leisten bis auf Anlage mit der Auflagefläche 16 einschnappen.

Aus Fig. 4 ist auch zu erkennen, wie die Linse 20 in gefaltetem Zustand um die Faltrippe 11 elastisch gelegt ist und im Transportkanal 7 eingedrückt festgehalten wird. Es ist ersichtlich, daß die Auflagefläche 16, die durch den nach oben schlitzförmig offenen Transportkanal 7 in zwei Teile unterbrochen ist, über große Radien 21 in die Seitenwände des Transportkanals übergehen. Zudem sind die beiden Teile der Auflagefläche 16 zur Horizontalen um einen Winkel 22 von ca. 30° und gleichzeitig in Richtung auf den Transportkanalboden geneigt, wodurch die Falt-Einschiebbewegung der Linse wesentlich erleichtert wird. Auch ist erkennbar, daß der Halteteil 5 durch seine Auflagenflächenteile und Transportkanalschlitz in Art einer Matritze und die Klappe mit der Faltrippe in Art einer Patritze eines Biege- oder Tiefziehwerkzeugs arbeitet.

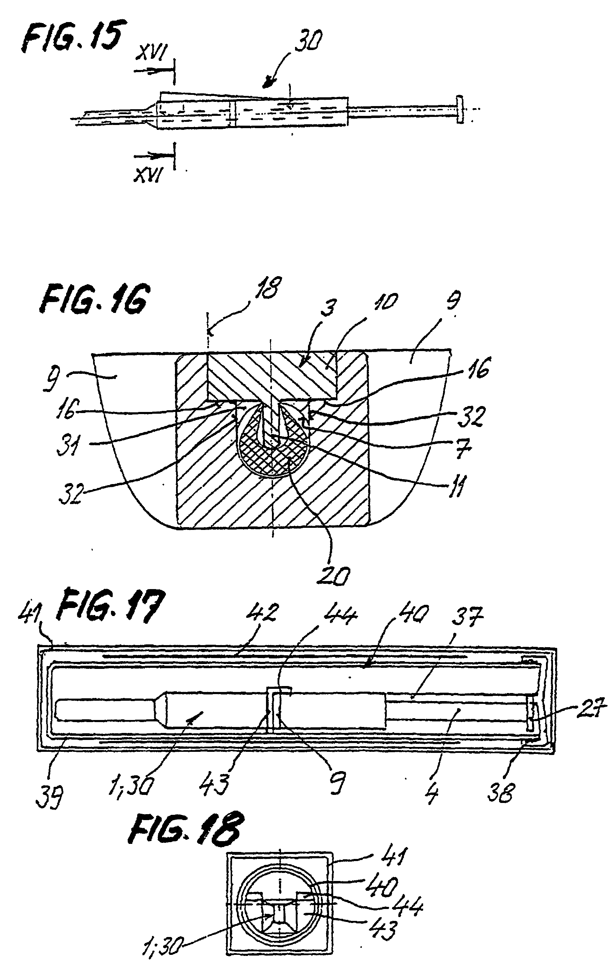

In Fig. 6 bis 14 sind die drei Einzelteile eines Injektors in zweiter Ausführungsform einzeln genau dargestellt.

So zeigen Fig. 6 bis 9 einen Injektorkörper, dessen Halteteil 5, wie beim vorhergehenden Beispiel nach Fig. 1 bis 5 rechteckig ausgebildet ist, während sein Einführrohr 6 als auch der Transportkanal 7 einen runden Querschnitt aufweisen. Des weiteren ist in dieser Ansicht die Anordnung der Lagerbohrungen 13 zu erkennen sowie von zwei Indexierpunkten, u.zw. einem ersten Indexierpunkt 24 für die Festlegung der Klappe mit auf der ungefalteten Linse aufliegender Faltrippe und ein zweiter Indexierpunkt 25 zum Festlegen der Position der Klappe in komplett eingedrücktem Zustand mit gefalteter, senkrecht eingeschobener Linse. Zudem ist insbesondere aus Fig. 6 erkennbar, daß die vordere Stirnseite des Einführrohres 6 mit der Austragöffnung 8 schräg angeordnet ist, so daß eine vordere Einführspitze 26 ausgebildet ist, die ein Einführen dieses Injektorteils durch den kleinen Schnitt in das Augeninnere wesentlich erleichtert.

Aus Fig. 8 bis 11 ist zu erkennen, daß der Klappenhebel breit ausgelegt ist und in eingedrücktem Zustand in die Oberfläche des Halteteils 5 komplett eingesenkt ist

Auch sind die Indexierpunkte 24 und 25 jeweils zu beiden Seiten erkennbar, wobei jedoch auch nur die Anordnung von Indexierpunkten an einer Seite reichen kann.

Aus Fig. 10 und 11 ist die Ausbildung der Klappe erkennbar, mit einem breiten Hebel 10 und einer schmalen Faltrippe 11, die hier direkt am Hebel 10 befestigt ist, ohne darüber befindlicher Druckplatte. Auch hier ist die Anordnung der Lager-Achsstummel 12, die hier als Noppen ausgebildet sind und der beiden Indexierpunkte 24 und 25, die ebenfalls noppenförmig zu beiden Seiten des Hebels vorgesehen sind, erkennbar.

Fig. 12 bis 14 zeigen einen Schieber, der in dieser Ausführungsform einen runden Querschnitt besitzt und an seiner äußeren Seite eine Druckplatte aufweist. An der Oberseite des Schiebers 4 ist eine Längsnut 28 eingebracht, deren Breite entsprechend der Breite der Faltrippe ausgelegt ist, so daß der Schieber trotz bis in den Transportkanal eingedrückter Faltrippe noch leicht längsverschieblich ist. Zudem ist an der vorderen Stirnseite des Schiebers 4 eine Schweifung 29 nach innen vorgesehen, einem Viertelkreis folgend, wodurch eine Anpassung an die Form der zu verschiebenden, gefalteten Linse vorhanden ist. Es ist zudem erkennbar, daß am Schieber drei Positionseinrichtungen vorhanden sind, und zwar erste Positionsnoppen 33, für die zurückgezogene Schieberposition, zweite Positionsnoppen 34 für die vorgeschobene Linsen-Position und dritte Position als Endanschlag 35 für die Stellung, in der die Linse aus dem Injektor komplett herausgeschoben ist.

Fig. 15 zeigt einen erfindungsgemäßen lnjektor 30 in ungefähren Maßstab 1:1, und zwar in der zweiten Ausführungsform nach Fig. 6 bis 14. Es ist zu erkennen, daß durch den extrem einfachen und aus nur drei Teilen bestehenden Aufbau des Injektors 30 die doch recht zarte Ausführung mühelos in einfachster Weise realisierbar und bedien- bzw. handhabbar ist.

Des weiteren zeigt Fig. 16 einen Schnitt XVI-XVI aus Fig. 15, ähnlich wie die Darstellung nach Fig. 4, mit eingedrückter Klappe 3. Hier ist der Hebel 10 der Klappe 3 als durchgehendes Flachteil ausgebildet, an dessen unterer Vorderseite die Falt-rippe 11 angeordnet ist. Die Unterseite des Hebels 10 dient gleichzeitig als Eindrück-Tiefenanschlag und zwar in Zusammenwirkung mit der Auflagefläche 16 für die ungefaltete Linse. Der Transportkanal 7 ist rund ausgebildet und die Einbringöffnung 18 mündet hier über einen Falt- und Einbringschlitz 31 mit Vertikalwänden 32 tangential in den runden Transportkanal 7.

In Fig. 17 und 18 ist ein verpackter Injektor 1, 30 darge-stellt. Ein erfindungsgemäßer Injektor 1,30 ist in einem Transportbehälter 40 eingebracht, der sich wiederum in einem Verpackungskarton 41 befindet, in dem zudem ein Beipackzettel 42 mitverpackt ist

Es ist erkennbar, daß der Transportbehälter 40 aus einer Hülse 39 besteht, die durch einen Deckel 38 verschlossen ist. Hier ist eine Hülse 39 mit rundem Querschnitt vorgesehen. Sie kann jedoch auch einen rechteckigen Querschnitt aufweisen.

Im Inneren der Hülse 39 sind zwei quer zueinander beabstandete Platten 43 angeordnet, die so zueinander beabstaandet sind, daß der Halteteil 5 des Körpers leicht dazwischen einschiebbar ist. Die Halteplatten 43 dienen als Einschieb-Längsanschlag für den Injektor, der über seine Griffplatten 9 in Einschiebrichtung ansteht. Die Halteplatten 43 besitzen an ihrer freien Oberseite in Einschiebrichtung weisende Haken 44, die die Oberseite der Griffplatten 9 umgreifen und so den Injektor an der Wandung der Hülse 39 anliegend festhalten. Zudem ist am Deckel 38 ein axial weisender Dom 37 vorgesehen, der an der rückwärtigen Stimseite des Körper-Halteteils des Injektors ansteht, wodurch der Körper zwischen Halteplatten 43 und Dorn 37 nach Schließen des Deckels festgeklemmt ist. So kann auch durch unsachgemäßes Behandeln und eventuelles Fallenlassen des Transportbehälters ein ungewolltes Eindrücken des Schiebers und damit Auswerfen der ungefalteten Linse aus dem Injektor vermieden werden.

Statt des am Deckel 38 vorgesehenen Dornes 37 kann jedoch auch ein in der Zeichnung nicht dargestellter Abstandsclip verwendet werden. Dieser hat eine Länge gleich dem Abstand zwischen rückwärtiger Stirnfläche des Halteteils und der Druckplatte des Schiebers in der zurückgezogenen erster Schieberposition. Er kann ein geschlitztes Rohr sein, das leicht quer auf die Schiebestange auf- und abgeschoben werden kann. Zudem stützt sich dann die äußere Stirnfläche der Schieber-Druckplatte an der Innenseite des Deckels ab, wobei dann sich günstig auswirkt, wenn diese Innenseite mit einem sehr weichen Material wie Schaumstoff ausgelegt ist.

### BEZUGSZEICHENLISTE

- 1.: Injektor, erste Ausf.
- 2.: Körper
- 3.: Klappe
- 4.: Schieber
- 5.: Halteteil
- 6.: Einführrohr
- 7.: Transportkanal
- 8.: Austragöffnung
- 9.: Griffplatte
- 10.: Hebel
- 11.: Faltrippe
- 12.: Achsstummel
- 13.: Lagerbolzen
- 14.: Schnappschlitz
- 15.: Druckplatte
- 16.: Auflagefläche
- 17.: Stirnseite
- 18.: Einbringöffnung
- 19.: Auflageleiste
- 20.: Linse
- 21.: Radius
- 22.: Neigung
- 23.: ---
- 24.: Erster Indexierpunkt
- 25.: Zweiter Indexierpunkt
- 26.: Einführschlitz
- 27.: Druckplatte
- 28.: Längsnut
- 29.: Schweifung
- 30.: Injektor, zweite Ausf.
- 31.: Schlitz
- 32.: Wände
- 33.: Erste Positionsnoppen
- 34.: Zweite Positionsnoppen
- 35.: Endanschlag
- 36.: ---
- 37.: Dorn
- 38.: Decke
- 39.: Hülse
- 40.: Transportbehälter
- 41.: Verpackungskarton
- 42.: Beipackzettel
- 43.: Halteplatten
- 44.: Haken

## Patentansprüche

1. Injektor (1, 30) zum Implantieren/Einbringen einer zeitweilig gefalteten Intraokularlinse in die Linsenkapsel eines Auges, mit
- einem Körper (2), der aus einem dickeren Einbring- und Halteteil (5) und einem einführseitigen, dünneren Einführrohr (6) besteht und eine axiale Durchgangsöffnung als Transportkanal (7) besitzt,
- einem Schieber (4), der in dem Transportkanal (7) axial verschiebbar ist, und
- einer im Einbring- und Halteteil (5) quer und gleichzeitig symmetrisch zur Achse des Transportkanals (7) vorgesehenen Einbringöffnung (18) als Einbringkanal für die Linse, die mit der Transport-Durchgangsöffnung in Verbindung steht, und in der die ungefaltete Linse, für Aufbewahrung und Transport zusammen mit dem Injektor (1, 30) auf einer Auflagefläche (16) flach aufliegt und über eine längsmittig zur Linse sich erstreckende Halterippe (11) festgehalten wird,
**dadurch gekennzeichnet,**
- **dass** der aus Halteteil (5) und Einführrohr (6) bestehende Körper (2) einteilig ausgebildet ist,
- **daß** der Transportkanal (7) eine durchgehende Körperöffnung mit konstantem Querschnitt ist,
- **daß** die in der Einbringöffnung (18) befindliche Auflagefläche (16) gegenüber der Achse des Transportkanals (7) in Einbringrichtung nach oben versetzt innerhalb des Halteteiles (5) angeordnet ist, und über einen Einbringschlitz (31) mit gleicher Breite wie die maximale Breite des Transportkanals (7), in diesen übergeht,
- und **daß** die Halterippe eine sich plattenförmig radial erstreckende Faltrippe (11) ist, die durch die Einbringöffnung (18) bis in den Transportkanal (7) radial eindrückbar ist, derart, daß die Linse um die Faltrippe (11) gefaltet vollständig in den Transportkanal eingebracht wird.

2. Injektor nach Anspruch 1,
**dadurch gekennzeichnet, daß** der Injektor-Körper (5) insgesamt einen Rechteck-Querschnitt aufweist und daß auch der Transportkanal (7) rechteckig ist.

3. Injektor nach Anspruch 2,
**dadurch gekennzeichnet, daß** der Querschnitt des Transportkanals (7) für den Transport der gefalteten Linse, mit eingedrückt gehaltener Faltrippe (11) ausgelegt ist.

4. Injektor nach Anspruch 1,
**dadurch gekennzeichnet, daß** der Schieber (4) eine Stange durchgehend gleichen Querschnitts ist, an deren Oberseite eine Nut (28) vorgesehen ist, in die die eingedrückte Faltrippe (11), beim Verschieben des Schiebers (4) hineinreicht und daß die Schieberstimseite eine ca. Viertel-Zylinder-Schweifung (29) nach innen aufweist, angepaßt an den Rand der gefalteten Linse (20).

5. Injektor nach Anspruch 4,
**dadurch gekennzeichnet, daß** am Schieber (4) und Körper (2, 5) in Längs- bzw. Schieberichtung drei Positioniereinrichtungen vorgesehen sind und zwar für eine erste, zurückgezogene Schieber-Position (33), mit freigegebener Linsen-Eingabeöffnung, für eine zweite Position (34), mit in Nähe der vorderen Austragöffnung vorgeschobener, gefalteter Linse und für eine dritte Endanschlag-Position (35) mit aus dem Injektor (1, 30) voll ausgeschobener Linse (20).

6. lnjektor nach Anspruch 1,
**dadurch gekennzeichnet, daß** die Auflagefläche (16) für die ungefaltete Linse (20) aus zwei durch den Einführschlitz (31) getrennte Teilflächen besteht, die in einem Winkel von ca. 30° zur Horizontalen dachförmig nach innen in Richtung auf den Transportkanal (7) geneigt sind und über Radien (21) in die Seitenwände (32) des Transportkanals bzw. Einbring-Schlitzes übergehen.

7. Injektor nach Anspruch 6,
**dadurch gekennzeichnet, daß** zu beiden Längsseiten der Einbringöffnung (18), oberhalb der Auflageflächen (16), Auflageleisten (19) für Anschlagleisten der Faltrippe (11) vorgesehen sind.

8. Injektor nach Anspruch 1,
**dadurch gekennzeichnet, daß** die Faltrippe (11) am vorderen Ende eines an der Körperoberseite schwenkbar angelenkten Klapphebels (3, 10) vorgesehen ist.

9. Injektor nach Anspruch 8,
**dadurch gekennzeichnet, daß** an dem um ca. 180° umklappbaren Hebel (3, 10) der Faltrippe (11) zwei Kugel- oder Noppen-Indexiereinrichtungen (24, 25) vorgesehen sind in Verbindung mit Einrast-Vorkehrungen am Körper, zum Festlegen der Hebelwinkelposition beim aufliegenden Festhalten der ungefalteten Linse und beim eingedrückten Halten der gefalteten Linse.

10. Injektor nach Anspruch 9,
**dadurch gekennzeichnet, daß** der Hebel (10) in Höhe der Falt-rippe (11) bis auf annähernd Körperbreite zu einer Druckplatte (15) verbreitert ist, die in eingedrücktem Faltzustand auf der Auflagefläche (16) oder den Auflageleisten (19) sich und gleichzeitig die Faltrippe (11) radial abstützend.

11. Injektor nach Anspruch 1,
**dadurch gekennzeichnet, daß** zu beiden Seiten am Körper-Halteteil (5) ca. mittig je eine quer ausladende Griffplatte (9) angebracht ist.

12. Injektor nach Anspruch 1,
**dadurch gekennzeichnet, daß** der Injektorkörper (2) insgesamt einen runden Querschnitt aufweist, mit rundem Transportkanal und runder Schieberstange und daß der radiale Eingabe- und Faltschlitz (31) zwischen Linsen-Auflagefläche (16) und Transportkanal (7) tangential zur Transportkanalwandung verlaufende Längswände (32) aufweist.

13. Injektor nach den Ansprüchen 1 bis 12,
**dadurch gekennzeichnet, daß** alle drei Bestandteile des Injektors, nämlich Körper (2), Klappe (3) und Schieber (4), aus durchsichtigem Kunststoff z.B. durch Spritzgießen gefertigt sind und daß die drei Injektorteile durch einfaches Ineinanderschieben und -drücken zusammengesetzt und durch Schnappnoppen oder Einrastgelenke zusammengehalten werden.

## Claims

1. Injector (1, 30) for implanting/inserting a temporarily folded intraocular lens into the capsula of the lens of the eye, with
- a body (2) which consists of a thicker insertion and holding part (5) and a thinner injection tube (6) on the injection side and has a continuous axial opening as transporting channel (7),
- a slider (4)which is displaceable axially in the transporting channel (7), and
- an insertion opening (18) which is provided in the insertion and holding part (5) transversely and at the same time symmetrically in relation to the axis of the transporting channel (7) and serves as insertion channel for the lens and communicates with the continuous transporting opening, and in which the unfolded lens lies flat on a supporting surface (16) for storage and transport together with the injector (1, 30) and is held by means of a holding rib (11) extending longitudinally and centrally in relation to the lens,
**characterised**
- **in that** the body (2) consisting of the holding past (5) and the injection tube (6) is embodied in one piece,
- **in that** the transporting channel (7) is a continuous body opening with a constant cross-section,
- **in that** the supporting surface (16) located in the insertion opening (18) is arranged inside the holding part (5) offset upwards in the direction of insertion in relation to the axis of the transporting channel (7), and runs through an insertion slot (31) with the same width as the maximum width of the transporting channel (7) into the latter,
- and **in that** the holding rib is a folding rib (11) which extends radially in the form of a plate and can be pressed radially into the transporting channel (7) through the insertion opening (18) so that the lens is inserted completely into the transporting channel folded around the folding rib (11).

2. Injector according to claim 1, **characterised in that** the injector body (5) in all exhibits a rectangular cross-section and **in that** the transporting channel (7) is also rectangular.

3. Injector according to claim 2, **characterised in that** the cross-section of the transporting channel (7) is designed for transport of the folded lens, with the folding rib (11) held pressed in.

4. Injector according to claim 1, **characterised in that** the slider (4) is a rod with the same cross-section throughout, the upper side of which is provided with a groove (28) into which the pressed in folding rib (11) extends during the displacement of the slider (4), and **in that** the end side of the slider exhibits an inward curve (29) roughly following a quarter cylinder, matching the edge of the folded lens (20).

5. Injector according to claim 4, **characterised in that** three positioning devices are provided on the slider (4) and body (2, 5) in the longitudinal or sliding direction, namely for a first retracted slider position (33) with the lens insertion opening exposed, for a second position (34) with the folded lens slid forward close to the front delivery opening, and for a third end stop position (35) with the lens (20) slid completely out of the injector (1, 30).

6. Injector according to claim 1, **characterised in that** the supporting surface (16) for the unfolded lens (20) consists of two part surfaces which are separated by the insertion slot (31) and are inclined inwards in the direction of the transporting channel (7) in the shape of a roof at an angle of approx. 30° to the horizontal, and run via radii (21) into the side walls (32) of the transporting channel and insertion slot.

7. Injector according to claim 6, **characterised in that** supporting strips (19) for stop strips of the folding rib (11) are provided on both longitudinal sides of the insertion opening (18) above the supporting surfaces (16).

8. Injector according to claim 1, **characterised in that** the folding rib (11) is provided at the front end of a pivoting lever (3, 10) articulated pivotably on the upper side of the body.

9. Injector according to claim 8, **characterised in that** two ball or projection indexing devices (24, 25) are provided on the lever (3, 10) of the folding rib (11) pivotable through approx. 180°, in conjunction with detent means on the body, for determining the angular position of the lever when lying on and holding the unfolded lens and when pressed in and holding the folded lens.

10. Injector according to claim 9, **characterised in that** level with the folding rib (11) the lever (10) is widened to roughly the width of the body forming a thrust plate (15) which is supported on the supporting surface (16) or the supporting strips (19) when pressed in and simultaneously supports the folding rib (11) radially.

11. Injector according to claim 1, **characterised in that** a transversely extending grip plate (9) is provided on the body holding part (5) roughly in the middle on both sides.

12. Injector according to claim 1, **characterised in that** the injector body (2) exhibits a round cross-section overall, with a round transporting channel and round slider rod, and **in that** the radial insertion and folding slot (31) between the lens supporting surface (16) and the transporting channel (7) exhibits longitudinal walls (32) running tangentially to the transporting channel walls.

13. Injector according to claims 1 to 12, **characterised in that** all three components of the injector, namely the body (2), flap (3) and slider (4), are made of transparent plastic and **in that** the three injector parts are assembled together by simply sliding and pressing them into one another, and held together by snap projections or locking joints.

## Revendications

1. Injecteur (1, 30) destiné à implanter/introduire un cristallin artificiel plié temporairement dans le sac cristallinien d'un oeil, comportant
- un corps (2) qui se compose d'une pièce de maintien et d'introduction (5) plus épaisse et d'un tube d'insertion (6) plus mince du côté de l'insertion et qui possède une ouverture traversante axiale servant de canal de transport (7)
- un coulisseau (4) qui est mobile axialement dans le canal de transport (7), et
- une ouverture d'introduction (18) ménagée dans la pièce de maintien et d'introduction (5) transversalement et en même temps symétriquement à l'axe du canal de transport (7) et servant de canal d'introduction pour le cristallin qui est en liaison avec l'ouverture traversante de transport et dans lequel le cristallin non plié repose à plat sur une surface d'appui en vue du stockage et du transport conjointement avec l'injecteur (1, 30) et est maintenu par une nervure de maintien (11) s'étendant longitudinalement au milieu du cristallin,
**caractérisé en ce que**
le corps (2) se composant de la pièce de maintien (5) et du tube d'introduction (6) est conformé en une seule pièce,
le canal de transport (7) est une ouverture de corps ménagée de bout en bout et ayant une section constante,
la surface d'appui (16) se trouvant dans l'ouverture d'introduction (18) est disposée décalée vers le haut à l'intérieur de la pièce de maintien (5) par rapport à l'axe du canal de transport (7) et se transforme en celui-ci par une fente d'introduction (31) de largeur égale à la largeur maximale du canal de transport (7), et **en ce que**
la nervure de maintien est une nervure de pliage (11), s'étendant radialement en forme de plaque, laquelle peut être insérée radialement par l'ouverture d'introduction (18) jusque dans le canal de transport (7) de telle sorte que le cristallin est introduit dans le canal de transport en étant totalement plié autour de la nervure de pliage (11).

2. Injecteur selon la revendication 1, **caractérisé en ce que** le corps d'injecteur (5) a dans l'ensemble une section rectangulaire et **en ce que** le canal de transport est également rectangulaire.

3. Injecteur selon la revendication 2, **caractérisé en ce que** la section du canal de transport (7) destiné au transport du cristallin plié est dotée d'une nervure de pliage (11) maintenue insérée.

4. Injecteur selon la revendication 1, **caractérisé en ce que** le coulisseau (4) est une barre de même section de bout en bout, sur la face supérieure de laquelle est ménagée une gorge (28) jusqu'à laquelle la nervure de pliage insérée (11) s'étend lors du déplacement du coulisseau (4) et **en ce que** la face frontale du coulisseau a une cambrure (29) vers l'intérieur à peu près en quart de cylindre, adaptée au bord du cristallin plié (20).

5. Injecteur selon la revendication 4, **caractérisé en ce qu'**il est prévu sur le coulisseau (4) et le corps (2, 5) dans la direction longitudinale ou de coulissement trois dispositifs de positionnement c'est-à-dire pour une première position retirée (33) du coulisseau, dans laquelle l'ouverture d'entrée de cristallin est libérée, pour une deuxième position (34), dans laquelle le cristallin plié est déplacé à proximité de l'ouverture d'extraction avant, et pour une troisième position de butée d'extrémité (35) dans laquelle le cristallin (20) est totalement sorti de l'injecteur (1, 30).

6. Injecteur selon la revendication 1, **caractérisé en ce que** la surface d'appui (16) destinée au cristallin non plié (20) se compose de deux surfaces secondaires séparées par la fente d'insertion (31), lesquelles sont inclinées en forme de toit vers l'intérieur en direction du canal de transport (7) suivant un angle d'environ 30° par rapport à l'horizontale et se transforment par des rayons (21) en les parois latérales (32) du canal de transport ou de la fente d'introduction.

7. Injecteur selon la revendication 6, **caractérisé en ce qu'**il est prévu des baguettes d'appui (19) pour des baguettes de butée de la nervure de pliage (11) des deux côtés longitudinaux de l'ouverture d'introduction (18), au-dessus des surfaces d'appui (16).

8. Injecteur selon la revendication 1, **caractérisé en ce que** la nervure de pliage (11) est prévue à l'extrémité avant d'un levier repliable (3, 10) articulé sur la face supérieure du corps.

9. Injecteur selon la revendication 8, **caractérisé en ce qu'**il est prévu deux dispositifs d'indexage à billes ou à boutons (24, 25) sur le levier (3, 10), dépliable sur environ 180°, de la nervure de pliage (11) en liaison avec des moyens d'encliquetage sur le corps en vue de fixer la position angulaire du levier lors du maintien en appui du cristallin non plié et lors du maintien inséré du cristallin plié.

10. Injecteur selon la revendication 9, **caractérisé en ce que** le levier (10) s'étend au niveau de la nervure de pliage (11) jusqu'à atteindre à peu près la largeur du corps en direction d'une plaque d'appui (15) qui s'appuie radialement à l'état de pliage inséré sur la surface d'appui (16) ou les baguettes d'appui (19) et également sur la nervure de pliage (11).

11. Injecteur selon la revendication 1, **caractérisé en ce qu'**une plaque de préhension (9), saillant transversalement, est montée au milieu de chaque côté de la pièce de maintien de corps (5).

12. Injecteur selon la revendication 1, **caractérisé en ce que** le corps d'injecteur (2) a dans l'ensemble une section circulaire et comporte un canal de transport circulaire et une barre coulissante circulaire et **en ce que** la fente de pliage et d'entrée (31) présente des parois longitudinales (32) s'étendant tangentiellement à la paroi du canal de transport entre la surface d'appui (16) du cristallin et le canal de transport (7).

13. Injecteur selon les revendications 1 à 12, **caractérisé en ce que** les trois éléments constitutifs de l'injecteur, à savoir le corps (2), le volet (3) et le coulisseau (4) sont tous fabriqués en matière plastique transparente par exemple par moulage par injection et **en ce que** les trois pièces d'injecteur sont assemblées simplement en s'appuyant les unes sur les autres ou en pénétrant les unes dans les autres et sont maintenues ensemble par des boutons d'encliquetage ou des articulations de verrouillage.
